# EUROPEAN PATENT APPLICATION

(11) **EP 1 386 625 A1**
(43) Date of publication of application: **04.02.2004**
(21) Application number: 03077166.1
(22) Date of filing: 09.07.2003
(51) Int. Cl.: A61M 1/16, C02F 1/78, C02F 1/32, B01D 35/02, B01F 5/10

(54) **Waste liquid treatment apparatus for hemodialyzers**

(30) Priority: 31.07.2002 JP 2002223628
(71) Applicant: Air Liquide Santé (International), 75341 Paris Cedex 07 (FR)
(72) Inventor: Sun, Xulin, Tsukuba-shi, Ibaraki-ken (JP); Ishibashi, Junko, Tsukuba-shi, Ibaraki-ken (JP); Kimura, Kenichiro, Tokyo (JP)
(74) Representative: Conan, Philippe Claude

(57) **Abstract**

A dialysis waste liquid treatment apparatus is provided, in which the formation of plugs in lines can be restrained and in which COD and BOD of hemodialysis waste liquid can be effectively reduced, without requiring a wide space.
A waste liquid treatment apparatus for hemodialyzers, which comprises a transport pipe (3) for transporting dialysis waste liquid produced from a hemodialyzer, a bacteriostatic treatment tank (2) for restraining the propagation of bacteria caused by the dialysis waste liquid transported by said transport pipe and an ozone water ejection nozzle (8) for ejecting ozone water into said bacteriostatic treatment tank.

## Description

### Field of the Invention

The present invention relates to a waste liquid treatment apparatus for hemodialyzers.

### Prior Arts

Waste liquid of hemodialyzers is rich in nutrients such as grape sugar and its chemical oxygen demand (COD) and biochemical oxygen demand (BOD) are higher, and hence it is preferred to lower COD and BOD before the waste liquid is discharged to a sewer. Hemodialysis waste liquid can be subjected to biological treatment if equipment such as an activated sludge tank is disposed. Since a wide space is required for installation of an activated sludge tank, however, it is almost impossible to install an activated sludge tank in a clinic or the like which is in a tenant building positioned in an urban area or particularly at the center of a city. In the existing condition, accordingly, hemodialysis waste liquid produced in most of the clinic in an urban area is directly discharged to a sewerage or public water zone, as it is, not subjected to any special treatment, or after only pH thereof has been regulated.

As an apparatus for treating hemodialysis waste liquid, there has been hitherto known, for example, one disclosed in the official gazette of Japanese Patent Application Laid-open (KOKAI) No. 14,947/2001. This is an apparatus of effectively mixing dialysis waste liquid and ozone water in a tank. This apparatus is aimed at removing odors of dialysis waste liquid and decomposing nutrients contained in the dialysis waste liquid.

Since the solubility of ozone to water is lower and ozone in water is quickly decomposed, however, BOD and COD levels of dialysis waste liquid can not be sufficiently reduced only by mixing ozone water into the dialysis waste liquid so that the same waste liquid is treated.

Furthermore, a serious problem resides in such a point that products of bacteria (mainly aerobic bacteria) grow in lines on the way where dialysis waste liquid reaches a tank from a hemodialyzer so that plugs are formed to clog the lines. In order to remove such plugs and to maintain the lines, there is required a complicated work.

### Problems Sought for Solution by the Invention

It is therefore an object of the present invention to provide a waste liquid treatment apparatus for hemodialyzers, in which the formation of plugs in lines can be restrained. It is another object of the present invention to provide a waste liquid treatment apparatus for hemodialyzers, in which COD and BOD of hemodialysis waste liquid can be reduced, without requiring a wide space.

### Means for Solution of the Problems

A waste liquid treatment apparatus for hemodialyzers according to the present invention comprises a transport pipe for transporting dialysis waste liquid produced from a hemodialyzer, a bacteriostatic treatment tank for restraining the propagation of bacteria caused by the dialysis waste liquid transported by said transport pipe and an ozone water ejection nozzle for ejecting ozone water into said bacteriostatic treatment tank.

In the treatment apparatus according to the present invention, said transport pipe preferably retains the dialysis waste liquid substantially gas-tightly until the dialysis waste liquid transported thereby reaches said bacteriostatic treatment tank. And, said ozone water ejection nozzle is preferably constructed so as to eject ozone water to a dialysis waste liquid outlet of said transport pipe and exposed wall surfaces in said bacteriostatic treatment tank.

The treatment apparatus according to the present invention may comprise an ozone gas supply unit for supplying ozone gas to the dialysis waste liquid in said bacteriostatic treatment tank and an ultraviolet lamp for irradiating ultraviolet light to the dialysis waste liquid in said bacteriostatic treatment tank.

In the treatment apparatus according to the present invention, it is preferred that an organic substance decomposing treatment tank is disposed in the rear stage of said bacteriostatic treatment tank, an ozone gas supply unit is disposed for supplying ozone gas to the dialysis waste liquid in said organic substance decomposing treatment tank and an ultraviolet lamp is disposed so as to irradiate ultraviolet light to the dialysis waste liquid in said organic substance decomposing treatment tank.

The ultraviolet lamp is preferably disposed so that said ultraviolet light is irradiated also to bubbles formed on the liquid surface of said dialysis waste liquid due to the addition of said ozone gas.

In the treatment apparatus according to the present invention, a water cooler and an ozone water production unit for dissolving ozone into water to produce ozone water are preferably disposed in the front stage of said bacteriostatic treatment tank.

### Embodiments of the Invention

The present invention will be described in detail.

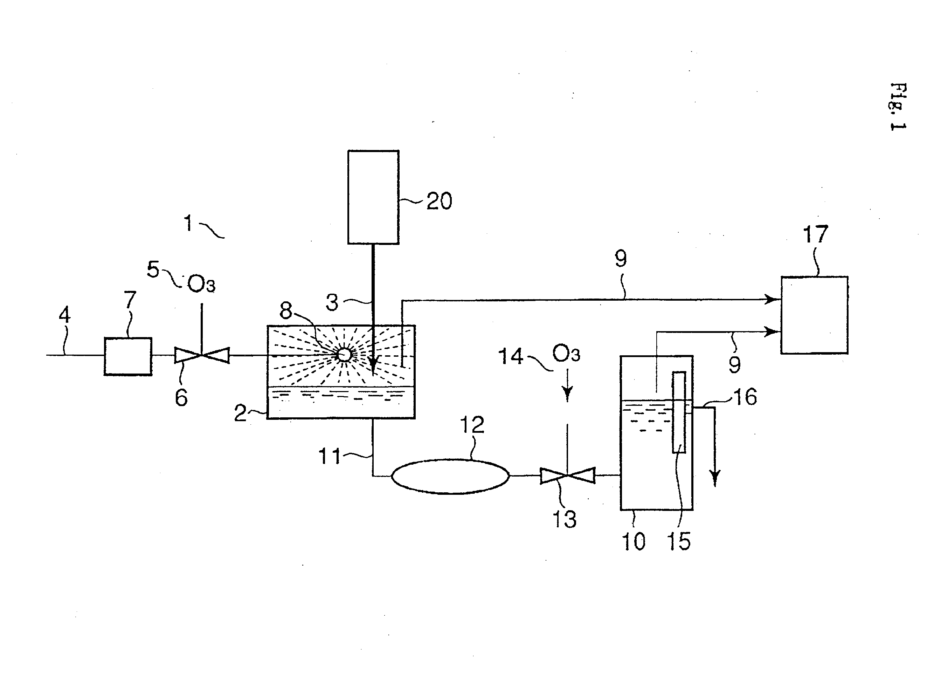

Fig. 1 is a block diagram showing the construction of a waste liquid treatment apparatus for hemodialyzers relating to one embodiment of the present invention. But, the treatment apparatus of the present invention is not limited thereto and can be variously modified for the purpose thereof.

The dialysis waste liquid treatment apparatus 1 illustrated in Fig.1 comprises as main treatment tanks a bacteriostatic treatment tank 2 for restraining the propagation of bacteria caused by dialysis waste liquid and an organic substance decomposing treatment tank 10 for decomposing organic substances contained in dialysis waste liquid.

Dialysis waste liquid discharged from a hemodialyzer 20 is transported to the bacteriostatic treatment tank 2 through a transport pipe 3. This hemodialyzer 20 comprises a dialysis solution producing unit and a patient monitoring unit. A dialysis waste liquid outlet of the transport pipe 3 is opened in a space above the liquid surface of the dialysis waste liquid stored in the bacteriostatic treatment tank 2. The dialysis waste liquid transported through this transport pipe 3 is preferably retained substantially gas-tightly (namely, it is almost prevented from being in contact with air) until it reaches the bacteriostatic treatment tank 2. In Fig. 1, in addition, a state is illustrated, in which one transport pipe 3 is extended from one hemodialyzer 20 to the bacteriostatic treatment tank 2. In a case where a plurality of hemodialyzers 20 are installed as in a clinic, however, transport pipes 3 are extended respectively or in connection from each of the hemodialyzers 20 to the bacteriostatic treatment tank 2.

In the front stage of the bacteriostatic treatment tank 2 is disposed a feed water pipe 4 for water for producing ozone water. Water used here may be city water or concentrated water (which is discharged as waste water) obtained in production of RO water, and there is no need of using relatively pure water such as RO water filtered using a reverse osmosis membrane (RO membrane) as described in the official gazette of Japanese Patent Application Laid-open (KOKAI) No. 14,947/2001. On the way of the feed water pipe 4 are disposed a cooler 7 and a venturi 6. The feed water pipe 4 is inserted in the bacteriostatic treatment tank 2 and has an ozone water ejection nozzle 8 disposed at an end thereof for ejecting ozone water. In addition, a pump may be disposed in front of the venturi 6 depending on the water pressure of the water used. In the bacteriostatic treatment tank 2 is disposed an exhaust pipe 9 for exhausting exhaust gas containing ozone to the atmosphere.

Water is cooled down by the cooler 7, and it is supplied in the venturi 6 with ozone from an ozone gas supply unit 5 so as to become ozone water. This ozone water is ejected from the ozone water ejection nozzle 8 into the bacteriostatic treatment tank 2. The ozone water ejection nozzle 8 is constructed so as to eject ozone water to the dialysis waste liquid outlet of the transport pipe 3 and exposed wall surfaces (side wall surfaces and roof wall surface) in the bacteriostatic treatment tank. By subjecting the dialysis waste liquid to an ozone treatment with ozone water in a state that it is almost prevented from being in contact with air, as mentioned above, the propagation of bacteria can be restrained and the formation of plugs can be effectively prevented in the transport pipe 3 and in the bacteriostatic treatment tank 2.

Although it is not always required to dispose the cooler 7, the cooler 7 is preferably disposed because it is preferred to cool down water so that the concentration of dissolved ozone is increased. The temperature of dialysis waste liquid is near to that (about 37°C) of a human body, and owing to the fact that the temperature of dialysis waste liquid is higher, the propagation of bacteria will be caused and besides any bad influence will be given upon the absorption rate of ozone gas in an organic substance decomposition tank of the rear stage. It is therefore preferred to dispose the cooler 7 so that ozone water having a lower temperature can be supplied.

Although the dialysis waste liquid treated in the bacteriostatic treatment tank 2 is biologically treated in an activated sludge tank, whereby COD and BOD thereof can be reduced, a wide installation space is required for the activated sludge tank, as mentioned above. In order to reduce COD and BOD of dialysis waste liquid in the treatment apparatus according to the present invention, furthermore, an organic substance decomposing treatment tank 10 is preferably disposed in addition to the aforementioned construction.

Between the bacteriostatic treatment tank 2 and the organic substance decomposing treatment tank 10 is disposed a line 11 for the dialysis waste liquid, and on the way of the line 11 are disposed a pump 12 and a venturi 13. The dialysis waste liquid treated in the bacteriostatic treatment tank 2 is transported through the line 11 by the pump 12 and supplied in the venturi 13 with ozone gas from an ozone gas supply unit 14, and it is thereafter fed into the organic substance decomposing treatment tank 10. In the organic substance decomposing treatment tank 10 is disposed an ultraviolet lamp 15 such as a low-pressure mercury lamp for irradiating ultraviolet light to the dialysis waste liquid.

In order to supply ozone gas to the dialysis waste liquid in the organic substance decomposing treatment tank 10, in addition, a diffuser may be disposed in the same organic substance decomposing treatment tank 10, whereby ozone gas from the ozone gas supply unit is caused to bubble.

In the organic substance decomposing treatment tank 10, organic substances are quickly decomposed by the actions of ozone gas and ultraviolet light, whereby COD and BOD thereof can be effectively reduced. In a hemodialyzer, by the way, disinfection is often performed using a disinfectant containing acetic acid or peracetic acid. While these substances will increase COD of dialysis waste liquid, they can be also effectively decomposed in the organic substance decomposing treatment tank 10. Since ozone is decomposed by the irradiation of ultraviolet light, the ozone concentration in exhaust gas can be reduced. When it is tried to treat dialysis waste liquid by using only ozone gas in the organic substance decomposing treatment tank 10, bubbles are formed and hence the dialysis waste liquid occasionally overflows the organic substance decomposing treatment tank 10. By irradiating ultraviolet light to these bubbles during treatment of the dialysis waste liquid with ozone gas, however, the formation of bubbles can be controlled. In such a manner that ultraviolet light can be irradiated not only to the dialysis waste liquid but also to bubbles formed on the liquid surface thereof, accordingly, the ultraviolet lamp 15 is preferably arranged, as shown in Fig.1, so as to come out of the liquid surface of the dialysis waste liquid. In addition, a plurality of ultraviolet lamps may be disposed. The dialysis waste liquid treated in the organic substance decomposing treatment tank 10 to reduce COD and BOD thereof can be discharged from a drainage port 16 to a sewerage.

In addition, it is conceived that a method of directly irradiating ultraviolet light to bubbles in the treatment of dialysis waste liquid with ozone gas to control the formation of bubbles can be applied to not only the dialysis waste liquid but also to another waste liquid which bubbles up in the ozone gas treatment thereof.

Also in the organic substance decomposing treatment tank 10 is disposed an exhaust pipe 9 for exhausting exhaust gas containing ozone to the atmosphere. After the exhaust gas containing ozone, which has been exhausted from the bacteriostatic treatment tank 2 and the organic substance decomposing treatment tank 10 through the exhaust pipes 9, is subjected to a treatment of ozone in an exhaust gas treatment unit 17 packed with activated carbon, a catalyst or the like, it will be released to the atmosphere. In addition, the exhaust gas treatment unit 17 may be one of performing the thermal decomposition of ozone gas.

Although the treatments of dialysis waste liquid with ozone gas and ultraviolet light are performed, as shown in Fig.1, in the organic substance decomposing treatment tank 10 disposed separately from the bacteriostatic treatment tank 2, it may be devised to subject the dialysis waste liquid in the bacteriostatic treatment tank 2 to the treatments with ozone gas and ultraviolet light.

In order to perform the treatment of dialysis waste liquid more effectively, furthermore, additives such as hydrogen peroxide may be added to the ozone water.

In the waste liquid treatment apparatus for hemodialyzers according to the present invention having such a construction as mentioned above, the formation of plugs in lines, which has hitherto been a problem sought for solution, can be restrained. And further, it is possible to effectively reduce COD and BOD, without requiring a wide space, as in a case where an activated sludge tank is installed.

In the next place, there will be described preliminary experiments performed until the dialysis waste liquid treatment apparatus according to the present invention is completed.

### Experiment 1

Dialysis solution used in hemodialysis is usually a mixed solution containing grape sugar and a hydrogencarbonate salt (such as sodium hydrogencarbonate) as a buffer and the like, and dialysis waste liquid contains proteins introduced from the blood of a patient in addition to such a mixed solution.

As for artificially prepared hemodialysis waste liquid (AHW) which was treated with ozone water, OD (opaque degree) and CFU (colony formation unit) thereof were measured to investigate the bacteriostatic effect of ozone water.

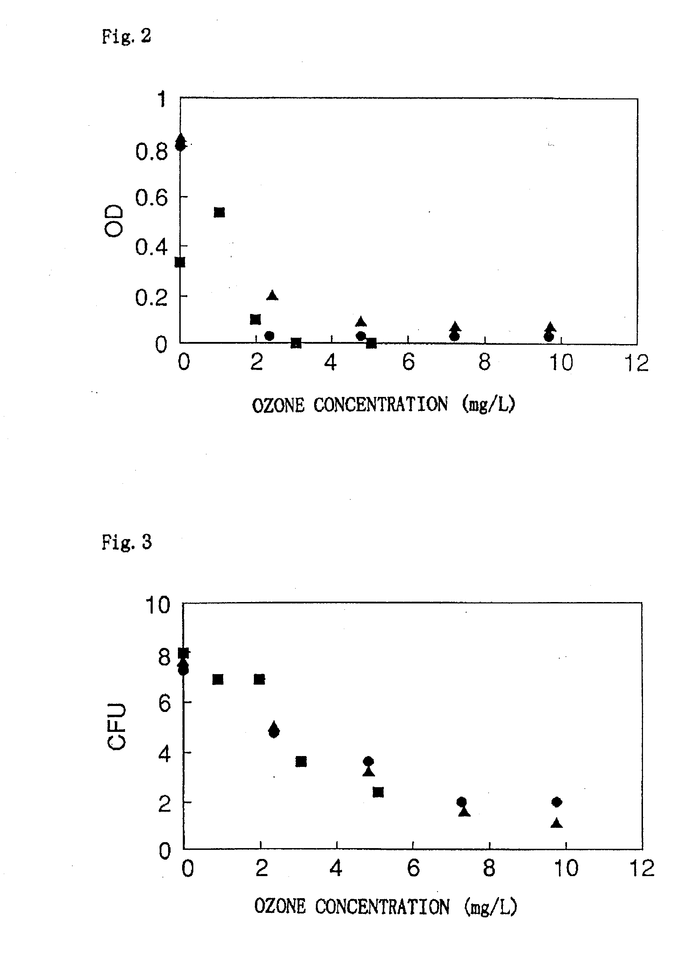

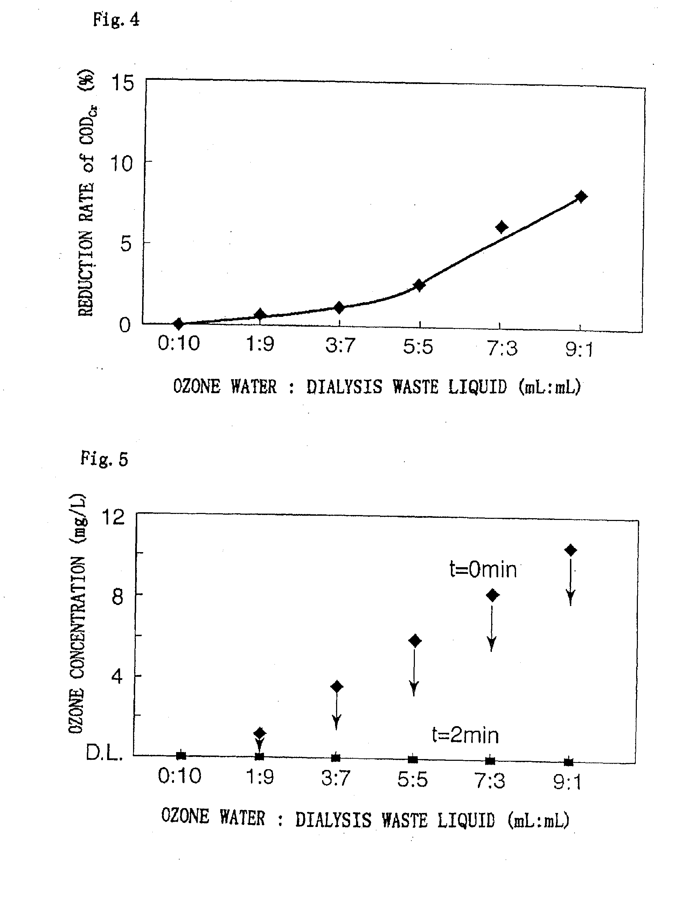

At first, bacteria were cultivated in AHW. 2mL of this solution were mixed with 8mL of ozone water having various concentrations and the resulting mixed liquid was left until ozone therein was not detected, and this was used as a sample. Ozone concentration was measured using indigo blue in accordance with the standards of AWWA (American Waste Water Association). After 100 µL of this sample were added in 5mL of Muller Hinton broth and incubated for 48 hours, the opaque degree (OD) thereof at a wavelength of 680nm was measured (Fig.2). Furthermore, 1mL of the sample was incubated on Petrifilm™ (for use in general measurement of the number of living bacteria, made by 3M Co.) for 48 hours, and CFU thereof was then measured (Fig.3). Experiments were respectively performed three times. In the graphs, respective measurement results are represented by marks different in shape.

From any results, it was revealed that the higher the ozone concentration was, the more the propagation of bacteria could be restrained.

### Experiment 2

The same AHW as used in Experiment 1 was respectively treated with water and ozone water, and both the treated liquids were compared by visual observation. A sample A was prepared by mixing 50mL of ozone water having a concentration of 10mg/L (10ppm) and 25mL of AHW containing bacteria and a sample B was prepared by mixing 50mL of water and 25mL of AHW containing bacteria, and both of them were observed after 5 days. As a result, the sample A was transparent and the sample B was opaque. Accordingly, it was also visually confirmed that the ozone-containing water restrained the propagation of bacteria.

### Experiment 3

Then, the relationship of the reduction rate of COD to the concentration of ozone water was investigated in the ozone water treatment of hemodialysis waste liquid.

Here, COD values measured using potassium bichromate as an oxidizing agent are designated by COD_{Cr}. Since acetic acid can be also detected in a measuring method using potassium bichromate, large values are obtained as compared with COD values (COD_{Mn}) measured using potassium permanganate as an oxidizing agent. The reason is that potassium bichromate oxidizes more kinds of organic substances than potassium permanganate oxidizes. Since most of the organic substances contained in dialysis waste liquid are detected as BOD, values approximate to BOD rather than COD_{Mn} are obtained for COD_{Cr}.

Ozone water having an ozone concentration of 11.7mg/L and AHW were mixed at various ratios, and COD_{Cr} of the respective resulting mixtures was measured. The reduction rate of this COD_{Cr} value represented by percentage as compared with COD_{Cr} in a case of only AHW was given in Fig.4. As this result, it was revealed that the higher the ozone concentration was, the more COD_{Cr} was reduced.

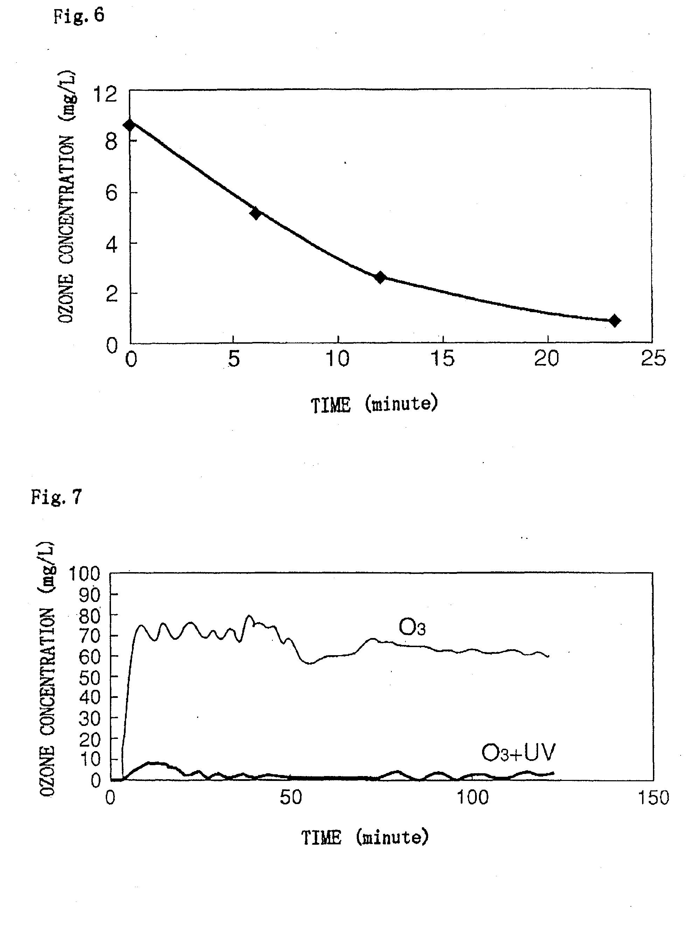

On the same samples as the aforementioned samples, the ozone concentration in the solution was measured at 2 minutes after the reaction was started. In all the samples, ozone was not detected (Fig.5). The hourly change of the concentration of ozone dissolved in pure water was given for comparison in Fig.6.

From the results of Fig.6 and Fig.5, it can be seen that ozone dissolved in pure water is decomposed within a short period of time and in a case where ozone water is mixed in dialysis waste liquid, in which organic substances exist, ozone is consumed within 2 minutes.

From the results of Experiments 1 ~ 3, it can be seen that if a region where dialysis waste liquid is in contact with air is not formed as wider as possible between the dialyzer 20 and the bacteriostatic treatment tank 2 and most of the bacteria are caused to become extinct by performing the ozone treatment of dialysis waste liquid with ozone water in the bacteriostatic treatment tank 2, as in the treatment apparatus of the present invention, the propagation of bacteria can be restrained and the formation of plugs can be effectively prevented. It is therefore conceived that it is effective to adopt a nozzle system, whereby ozone water is ejected, as it is, to the dialysis waste liquid outlet of the transport pipe 3 and the exposed wall surfaces in the bacteriostatic treatment tank 2, not directly mixed into the dialysis waste liquid.

In the present invention, it is preferred to supply ozone to cooling water so that the ozone concentration in ozone water is enhanced, as shown in Fig.1. The solubility of ozone to water is disclosed, for example, in Ozone in Water Treatment: Application and Engineering, edited by Bruno Langlais, David Reckhow & Deborah R. Brink, Lewis Publishers, 1991, p113. According to this literature, the ratio of the concentration (mg/L) of ozone dissolved in water to the ozone concentration (mg/L) in a gas to be added is 0.12 at 35°C, 0.21 at 20°C and 0.29 at 14.5°C. Accordingly, the lower the temperature of water is, the more the absorption rate of ozone is increased, wherein ozone can be more effectively used, and hence it is preferred in the present invention to cool down water to below 20°C. If cool ozone water is utilized to lower the temperature of dialysis waste liquid from 37°C to below 20°C, the absorption rate of ozone in the ozone gas supply unit 14 of the rear stage can be increased nearly twice.

### Experiment 4

The influence of ultraviolet irradiation was investigated in the treatment of dialysis waste liquid using ozone.

At first, 100mg/L of ozone gas were supplied and mixed into 5L of AHW. Then, there was measured the ozone concentration in exhaust gas discharged in a case where ultraviolet irradiation with the low-pressure mercury lamp was performed on the resulting mixture and in a case where it was not performed thereon. The results were given in Fig.7. From these results, it was revealed that ozone was contained in the exhaust gas in a case where ultraviolet irradiation was not performed, but ozone was not practically contained therein in a case where it was performed.

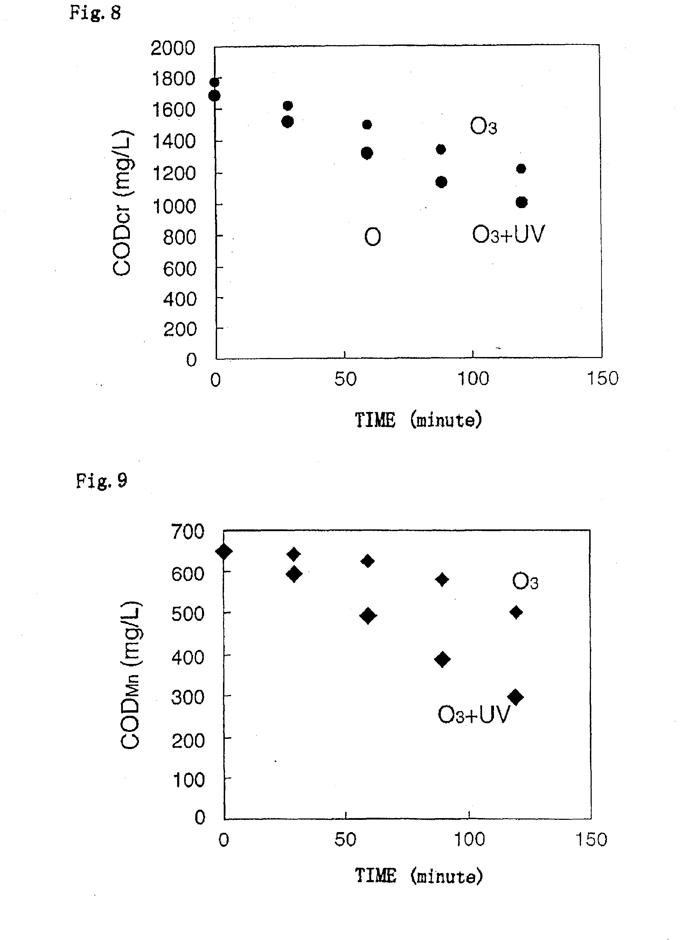

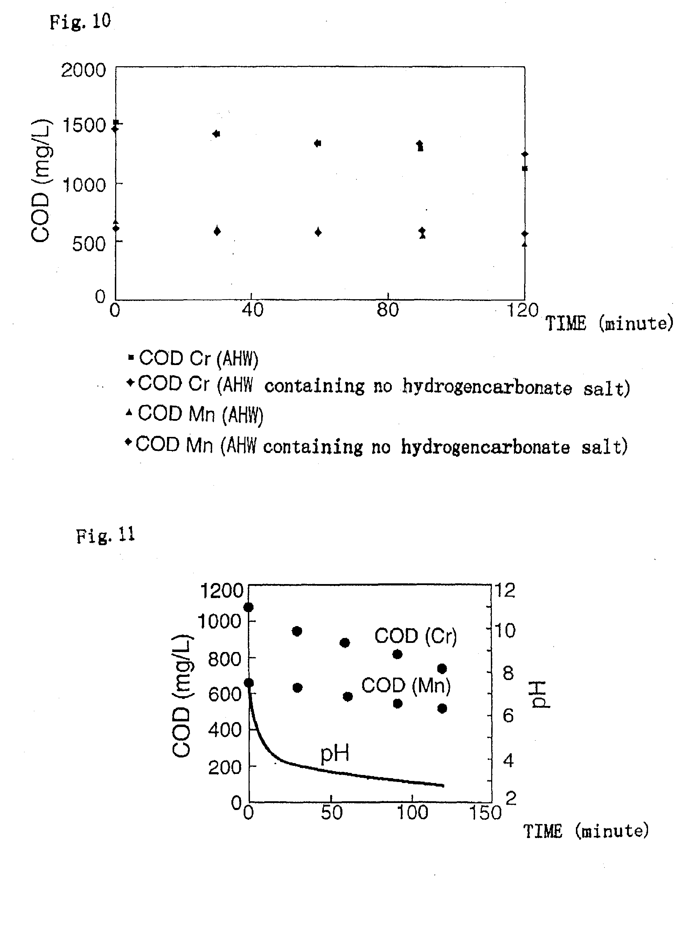

Then, the influence of ultraviolet irradiation upon COD of the waste liquid was investigated. COD_{Cr} (Fig.8) and COD_{Mn} (Fig.9) were respectively measured on 5L of AHW supplied with 100mg/L of ozone gas, to which ultraviolet light was irradiated by the low-pressure mercury lamp and to which it was not irradiated. From these graphs, it was revealed that both COD_{Cr} and COD_{Mn} were reduced more quickly on AHW, to which ultraviolet light was irradiated.

From these experiments, it was revealed that although a large amount of bubbles were formed when ozone gas was supplied to dialysis waste liquid, without irradiating ultraviolet light thereto, the formation of bubbles was immediately ceased when ozone gas was supplied to the dialysis waste liquid, while irradiating ultraviolet light thereto.

Here, it is preferred for obtaining a considerable treatment rate that the concentration of ozone gas to be supplied in the venturi 13 of Fig.1 is made more than 50mg/L.

### Experiment 5

It is generally said that a hydrogencarbonate salt contained in a dialysis solution as a buffer has any bad influence upon the treatment of waste liquid with ozone. Thus, the influence of a hydrogencarbonate salt upon the treatment of waste liquid with ozone was investigated. Concretely, 100mg/L of ozone gas were supplied respectively to artificially prepared dialysis waste liquid (AHW) which was the same as used in Experiments 1~4 and to dialysis waste liquid prepared by using dialysis solution containing no hydrogencarbonate salt, and the hourly changes of COD_{Cr} and COD_{Mn} of the respective resulting mixtures were investigated. The results are given in Fig. 10. From this graph, it was revealed that the hydrogencarbonate salt had no bad influence upon the treatment of dialysis waste liquid with ozone because the reductions of COD_{Cr} and COD_{Mn} were sooner in the dialysis waste liquid containing the hydrogencarbonate salt rather than one containing no hydrogencarbonate salt.

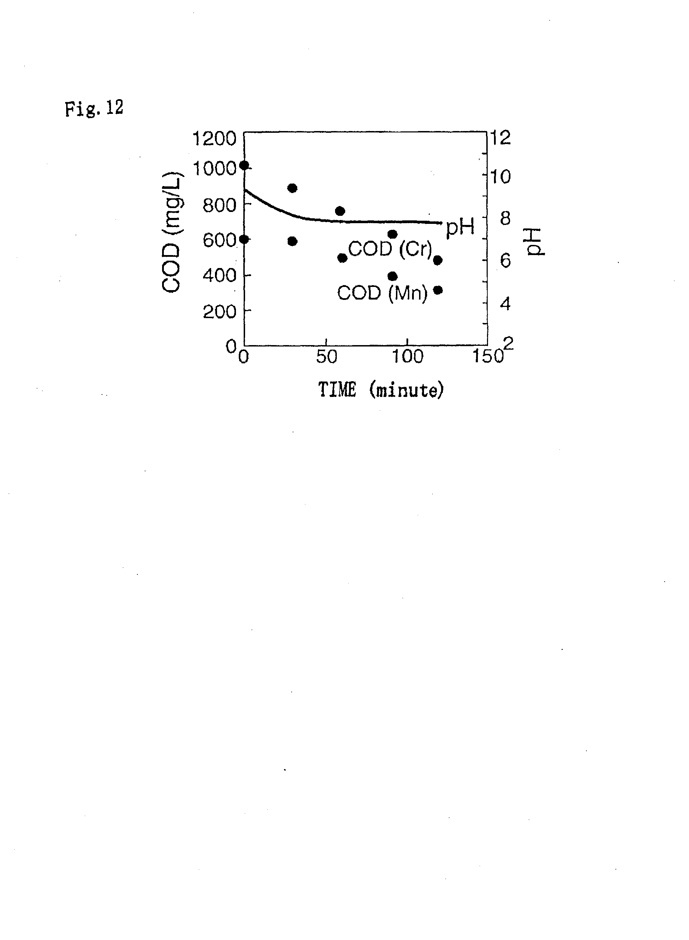

Then, the influence of a hydrogencarbonate salt in the ozone treatment of dialysis waste liquid was investigated by using grape sugar which is a main causing substance for COD in the dialysis waste liquid. There were prepared an aqueous solution containing 1g/L of grape sugar and an aqueous solution containing 1g/L of grape sugar and 25.2g/L of a hydrogencarbonate salt. Then, 100mg/L of ozone gas were supplied to these aqueous solutions, and the hourly changes of COD_{Cr}, COD_{Mn} and pH of the respective resulting solutions were investigated (Fig. 11 and Fig.12). In a case where the hydrocarbonate salt was not contained, as the results, pH was lowered with the change of time (Fig. 11), and in a case where the hydrocarbonate salt was contained, pH was kept constant due to its buffering action (Fig. 12). In both the cases, however, COD was reduced with the lapse of time. Accordingly, it was revealed that the hydrogencarbonate salt had no bad influence upon the ozone treatment of the waste liquid and COD was lowered within a short period of time rather in the solution containing the hydrogencarbonate salt.

### Effects of the Invention

According to the dialysis waste liquid treatment apparatus of the present invention, as has been described above in detail, the formation of plugs in lines can be restrained. And further, COD and BOD of hemodialysis waste liquid can be effectively reduced, without requiring a wide space.

### Brief Description of the Drawings

Fig. 1 is a view showing the construction of the waste liquid treatment apparatus for hemodialyzers relating to one embodiment of the present invention.
Fig.2 is a graph representing the opaque degree (OD) exhibiting the propagation of bacteria in a case where AHW was treated with ozone water having various concentrations.
Fig.3 is a graph exhibiting the number of existing bacteria in a case where AHW was treated with ozone water having various concentrations.
Fig.4 is a graph representing the relationship between the ozone concentration and the reduction rate of COD_{Cr} in the ozone water treatment of AHW.
Fig.5 is a graph representing the relationship between the initial ozone concentration and the ozone concentration after two minutes in the ozone water treatment of AHW.
Fig.6 is a graph representing the hourly change of the concentration of ozone dissolved in pure water.
Fig.7 is a graph representing the hourly changes of the ozone concentration in exhaust gas in a case where ultraviolet light was irradiated in the ozone water treatment of AHW and in a case where it was not irradiated.
Fig.8 is a graph representing the hourly changes of COD_{Cr} of waste liquid in a case where ultraviolet light was irradiated in the ozone water treatment of AHW and in a case where it was not irradiated.
Fig.9 is a graph representing the hourly changes of COD_{Mn} of waste liquid in a case where ultraviolet light was irradiated in the ozone water treatment of AHW and in a case where it was not irradiated.
Fig. 10 is a graph representing the influence of a hydrogencarbonate salt upon the ozone treatment of dialysis waste liquid.
Fig. 11 is a graph representing the hourly changes of COD_{Cr}, COD_{Mn} and pH in the ozone treatment of an aqueous grape sugar solution. And
Fig. 12 is a graph representing the hourly changes of COD_{Cr}, COD_{Mn} and pH in the ozone treatment of a solution containing grape sugar and a hydrogencarbonate salt.

### Description of Reference Numerals

1 - dialysis waste liquid treatment apparatus, 2 - bacteriostatic treatment tank, 3-transport pipe, 4 - feed water pipe, 5 - ozone gas supply unit, 6 - venturi, 7 - cooler, 8 - ozone water ejection nozzle, 9 - exhaust pipe, 10 - organic substance decomposing treatment tank, 11 - line, 12 - pump, 13 - venturi, 14 - ozone gas supply unit, 15-ultraviolet lamp, 16 - drainage port, 17 - exhaust gas treatment unit, 20 - dialyzer.

## Claims

1. A waste liquid treatment apparatus for hemodialyzers, which comprises a transport pipe for transporting dialysis waste liquid produced from a hemodialyzer, a bacteriostatic treatment tank for restraining the propagation of bacteria caused by the dialysis waste liquid transported by said transport pipe and an ozone water ejection nozzle for ejecting ozone water into said bacteriostatic treatment tank.

2. A waste liquid treatment apparatus for hemodialyzers, according to claim 1, in which said transport pipe retains the dialysis waste liquid substantially gas-tightly until the dialysis waste liquid transported thereby reaches said bacteriostatic treatment tank.

3. A waste liquid treatment apparatus for hemodialyzers, according to claim 1, in which said ozone water ejection nozzle is constructed so as to eject ozone water to a dialysis waste liquid outlet of said transport pipe and exposed wall surfaces in said bacteriostatic treatment tank.

4. A waste liquid treatment apparatus for hemodialyzers, according to anyone of claims 1 to 3, which comprises an ozone gas supply unit for supplying ozone gas to the dialysis waste liquid in said bacteriostatic treatment tank and an ultraviolet lamp for irradiating ultraviolet light to the dialysis waste liquid in said bacteriostatic treatment tank.

5. A waste liquid treatment apparatus for hemodialyzers, according to anyone of claims 1 to 3, in which an organic substance decomposing treatment tank is disposed in the rear stage of said bacteriostatic treatment tank, an ozone gas supply unit is disposed for supplying ozone gas to the dialysis waste liquid in said organic substance decomposing treatment tank and an ultraviolet lamp is disposed so as to irradiate ultraviolet light to the dialysis waste liquid in said organic substance decomposing treatment tank.

6. A waste liquid treatment apparatus for hemodialyzers, according to claim 4 or 5, in which said ultraviolet lamp is disposed so that said ultraviolet light is irradiated also to bubbles formed on the liquid surface of said dialysis waste liquid due to the addition of said ozone gas.

7. A waste liquid treatment apparatus for hemodialyzers, according to anyone of claims 1 to 6, in which a water cooler and an ozone water production unit for dissolving ozone into water to produce ozone water are disposed in the front stage of said bacteriostatic treatment tank.
